# EUROPEAN PATENT APPLICATION

(11) **EP 4 736 909 A1**
(43) Date of publication of application: **06.05.2026**
(21) Application number: 24856200.1
(22) Date of filing: 23.07.2024
(51) Int. Cl.: A61M 5/142

(54) **DRUG SOLUTION ADMINISTRATION DEVICE AND DRUG SOLUTION ADMINISTRATION SYSTEM**

(30) Priority: 23.08.2023 JP 2023135313
(71) Applicant: Terumo Kabushiki Kaisha, Tokyo 151-0072 (JP)
(72) Inventor: UCHIYAMA, Joji, Ashigarakami-gun, Kanagawa 259-0151 (JP); SEKIGUCHI, Shota, Ashigarakami-gun, Kanagawa 259-0151 (JP)
(74) Representative: TBK
(86) International application number: PCT/JP2024/026241
(87) International publication number: WO 2025/041506

(57) **Abstract**

A drug solution administration system (10) includes a drug solution administration device (100) that is detachably mounted to a holder (20). A casing (120) constituting the drug solution administration device has a bottom outer surface (126) that faces a holder inner surface (26) when the drug solution administration device is mounted to the holder. A flow path forming member (166) is accommodated in a recessed groove (132) of the bottom outer surface. The flow path forming member has an opening (172) and a hollow interior (170). The hollow interior forms a drug solution flow path (210) within the recessed groove. A drug solution discharged from a reservoir (102) in the casing flows along the drug solution flow path toward a cannula (66).

## Description

### Technical Field

The present invention relates to a drug solution administration device and a drug solution administration system for administering a drug solution to a living subject.

### Background Art

A portable drug solution administration system includes a cannula, a reservoir, and a pump unit. In the drug solution administration systems disclosed in US 11,291,764 and WO 2019/008562 A, the cannula, the reservoir, and the pump unit are accommodated in the same casing. A drug solution is stored in the reservoir.

When using a drug solution administration system configured as described above, the user first fixes the casing to the body of a patient by adhering it thereto. Next, the user causes the distal end of the cannula to protrude from the holder and indwell in the body of the patient. Thereafter, the pump unit discharges the drug solution from the inside of the reservoir to the outside of the reservoir. The drug solution discharged from the reservoir is administered into the body of the patient through the cannula.

In US 11,291,764, as illustrated in Fig. 1, the reservoir and the cannula are connected to each other via tubes (reference signs TU1 and TU2). In WO 2019/008562 A, as illustrated in Fig. 10G, the reservoir and the cannula are connected to each other via a joint (reference sign 548). As understood from Fig. 1 of US 11,291,764 and Fig. 10G of WO 2019/008562 A, the tubes and the joint are accommodated inside the casing.

### Summary of Invention

In the configurations disclosed in US 11,291,764 and WO 2019/008562 A, as described above, it is necessary to accommodate the cannula, the reservoir, the pump unit, and the tubes (or the joint) in the casing. Therefore, it is not easy to downsize the drug solution administration device.

An object of the present invention is to solve the above-described problem.
(1) One aspect of the present invention is a drug solution administration device including: a reservoir that is filled with a drug solution; and a casing that accommodates the reservoir, wherein the casing has a bottom portion including: a bottom outer surface which faces a living subject when the drug solution is administered to the living subject, and a bottom inner surface which is a back surface of the bottom outer surface, the reservoir is arranged on the bottom inner surface, the bottom outer surface has a recessed groove which is recessed in a direction orthogonal to an axial direction of the casing and directed toward the bottom inner surface, the bottom outer surface includes a groove inner side surface and a ceiling surface which are inner surfaces of the recessed groove, the drug solution administration device includes a flow path forming member which is accommodated in the recessed groove and has a hollow interior, and the hollow interior of the flow path forming member forms, within the recessed groove, a drug solution flow path through which the drug solution flows.

According to this configuration, it is possible to supply the drug solution from the reservoir inside the casing to the drug solution flow path formed outside the casing. In this case, it is unnecessary to bridge a tube or the like from the reservoir to the cannula inside the casing. Therefore, it is unnecessary to accommodate a tube inside the casing. As a result, the casing can be downsized, thereby enabling the downsizing of the drug solution administration device.

Further, in assembling the drug solution administration device, it is easier to assemble the flow path forming member outside the casing than to additionally accommodate a tube inside the casing that has already accommodated the reservoir and the pump unit.

(2) In the drug solution administration device according to item (1) above, an entirety of the flow path forming member may be accommodated in the recessed groove in a thickness direction which is orthogonal to the axial direction of the casing and directed toward the bottom outer surface.

When the drug solution administration device is mounted to or detached from the holder, interference of the flow path forming member with the holder is avoided. Accordingly, the drug solution administration device can be smoothly mounted to or detached from the holder.

(3) In the drug solution administration device according to item (1) or (2) above, the reservoir may have a drug solution outlet from which the drug solution is discharged, the casing may have a communication hole which communicates with the drug solution outlet, and the drug solution outlet and the drug solution flow path may communicate with each other via the communication hole.

In this case, inside the casing, it is not necessary to establish communication between the drug solution outlet of the reservoir and the hollow interior of the flow path forming member via a tube or the like. Therefore, the number of components inside the casing is reduced, allowing for greater flexibility in the internal layout of the components in the casing. Furthermore, the downsizing and weight reduction of the drug solution administration device can also be achieved.

(4) In the drug solution administration device according to any one of items (1) to (3) above, when the drug solution administration device is viewed in a thickness direction which is orthogonal to the axial direction of the casing and directed toward the bottom outer surface, at least a portion of the drug solution flow path may overlap with the reservoir.

With this configuration, the width direction of the drug solution administration device, which is orthogonal to the axial direction and the thickness direction, can be reduced.

(5) In the drug solution administration device according to item (4) above, the reservoir may have a curved side surface which is arcuate and curves in a convex shape toward the flow path forming member, and when a direction toward the bottom outer surface in the thickness direction is defined as a downward direction, and a direction opposite to the downward direction and away from the bottom outer surface is defined as an upward direction, a position of the flow path forming member in the thickness direction may be between a lowermost portion of the reservoir and a central portion of the reservoir in the thickness direction.

In other words, when the drug solution administration device is viewed in the width direction which is orthogonal to the axial direction and the thickness direction, the flow path forming member may be positioned between the lowermost portion of the reservoir and the central portion of the reservoir in the thickness direction.

According to this configuration, the drug solution flow path can be arranged in close proximity to the curved side surface. Accordingly, dead space around the reservoir is reduced. As a result, the drug solution administration device can be downsized.

(6) In the drug solution administration device according to any one of items (1) to (5) above, a material of the flow path forming member may be resin.

Since the flow path forming member is not made of metal, the environmental burden can be reduced.

(7) In the drug solution administration device according to item (6) above, the flow path forming member may be transparent.

In this case, the hollow interior of the flow path forming member can be visually observed. Therefore, it is easy to confirm whether foreign matter is mixed in the drug solution within the drug solution flow path, whether the drug solution has entrained air, or the like.

(8) In the drug solution administration device according to any one of items (1) to (7) above, the drug solution flow path may extend in an axial direction of the reservoir, and the axial direction of the casing may coincide with the axial direction of the reservoir.

In this case, compared to a case where the drug solution flow path extends in another direction, the space required to form the drug solution flow path can be reduced. In other words, space-saving of the drug solution flow path is achievable.

(9) In the drug solution administration device according to item (8) above, a flow direction of the drug solution in the drug solution flow path may be opposite to a pushing direction on the drug solution stored inside the reservoir.

In this case, compared to a case where the pushing direction on the drug solution stored inside the reservoir coincides with the flow direction of the drug solution in the drug solution flow path, the length of the drug solution administration device in the axial direction can be reduced.

(10) In the drug solution administration device according to item (9) above, the flow path forming member may be substantially L-shaped.

According to this configuration, it is possible to change the direction of the drug solution discharged from the reservoir to a direction opposite to the pushing direction on the drug solution, while shortening the length of the drug solution flow path.

(11) Another aspect of the present invention is a drug solution administration system including: the drug solution administration device according to any one of items (1) to (10) above; and a holder that the drug solution administration device is detachably mounted to, wherein the holder has a cannula unit including a cannula to be indwelled in a body of a living subject, and the cannula unit is positioned in the holder between two end portions in the axial direction, and between two end portions in a width direction which is orthogonal to both the axial direction and a thickness direction, which is orthogonal to the axial direction of the casing and directed toward the bottom outer surface.

Since the cannula unit is not positioned at an edge portion of the holder, the entire edge portion of the holder is fixed to the body of the patient. Accordingly, the holder is less likely to be detached from the body. As a result, detachment of the cannula from the skin is avoided.

(12) In the drug solution administration system according to item (11) above, the cannula unit may have a first connecting portion, the flow path forming member may have: a second connecting portion which is detachably connected to the first connecting portion, and an opening which is formed on a side portion facing the ceiling surface of the recessed groove and communicates with the hollow interior, and the recessed groove and the hollow interior may form a drug solution flow path through which the drug solution discharged from the reservoir flows toward the cannula.

According to this configuration, by connecting the first connecting portion of the holder and the second connecting portion of the drug solution administration device, the lumen of the cannula can be brought into communication with the drug solution flow path of the drug solution administration device. Therefore, the drug solution in the reservoir can be administered to the living subject via the lumen of the cannula.

According to the present invention, it is unnecessary to accommodate a tube inside the casing, and thus, the casing can be downsized. As a result, the drug solution administration device can be downsized.

Further, in assembling the drug solution administration device, it is easier to assemble the flow path forming member outside the casing than to additionally accommodate a tube inside the casing that has already accommodated various members (such as the reservoir) and various mechanisms (such as the pump unit).

### Brief Description of Drawings

Fig. 1 is an overall schematic side view of a drug solution administration system according to an embodiment of the present invention.
Fig. 2 is an overall schematic perspective view of a holder constituting the drug solution administration system.
Fig. 3 is a schematic side sectional view of the drug solution administration system as viewed from a width direction.
Fig. 4 is a schematic plan view of a drug solution administration device as viewed from an upward direction, which is one direction extending in a thickness direction.
Fig. 5 is a sectional view taken along line V-V in Fig. 4.
Fig. 6 is a schematic perspective view of a first half constituting a casing of the drug solution administration device.
Fig. 7A is a schematic side view illustrating a position of a guide rib when the drug solution administration device is mounted to the holder. Fig. 7B is a schematic side sectional view of the drug solution administration system in the state illustrated in Fig. 7A, as viewed from the width direction.
Fig. 8A is a schematic side view illustrating a position of the guide rib when the drug solution administration device is relatively moved in a detachment direction with respect to the holder. Fig. 8B is a schematic side sectional view of the drug solution administration system in the state illustrated in Fig. 8A, as viewed from the width direction.
Fig. 9A is a schematic side view illustrating a position of the guide rib when the drug solution administration device moves obliquely with respect to the holder. Fig. 9B is a schematic side sectional view of the drug solution administration system in the state illustrated in Fig. 9A, as viewed from the width direction.
Fig. 10A is a schematic side view illustrating a position of the guide rib when the drug solution administration device further moves obliquely from the state illustrated in Fig. 9A with respect to the holder. Fig. 10B is a schematic side sectional view of the drug solution administration system in the state illustrated in Fig. 10A, as viewed from the width direction.

### Description of Embodiments

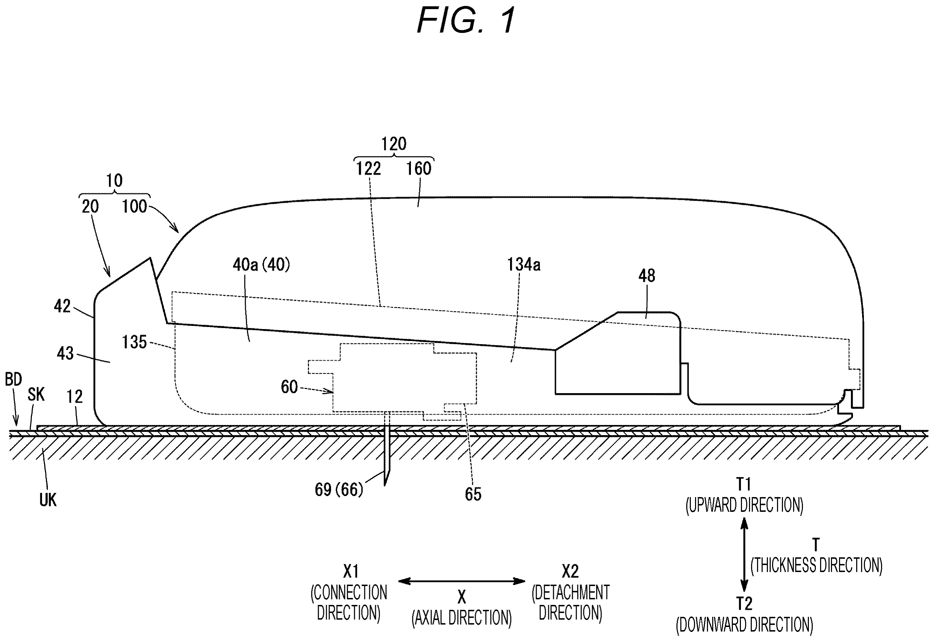

The term "living subject" in the present invention includes both human patients and animal patients; however, in the following description, a human patient will be exemplified as the living subject. The "user" refers to a person who uses a drug solution administration system 10 illustrated in Fig. 1 by attaching it to a body BD of a living subject. A typical example of the user is a patient, but the user is not limited to a patient.

Fig. 1 is an overall schematic side view of the drug solution administration system 10. The drug solution administration system 10 is used while being fixed to the body BD of the patient. The drug solution administration system 10 is, for example, fixed to a skin SK of the patient by an adhesive sheet 12 provided on a lower surface of a holder 20 that constitutes the drug solution administration system 10.

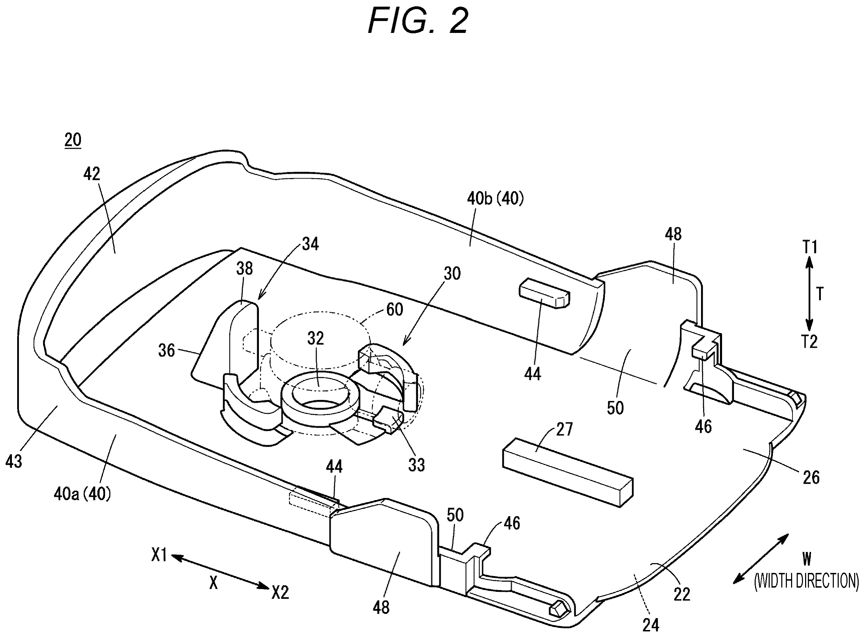

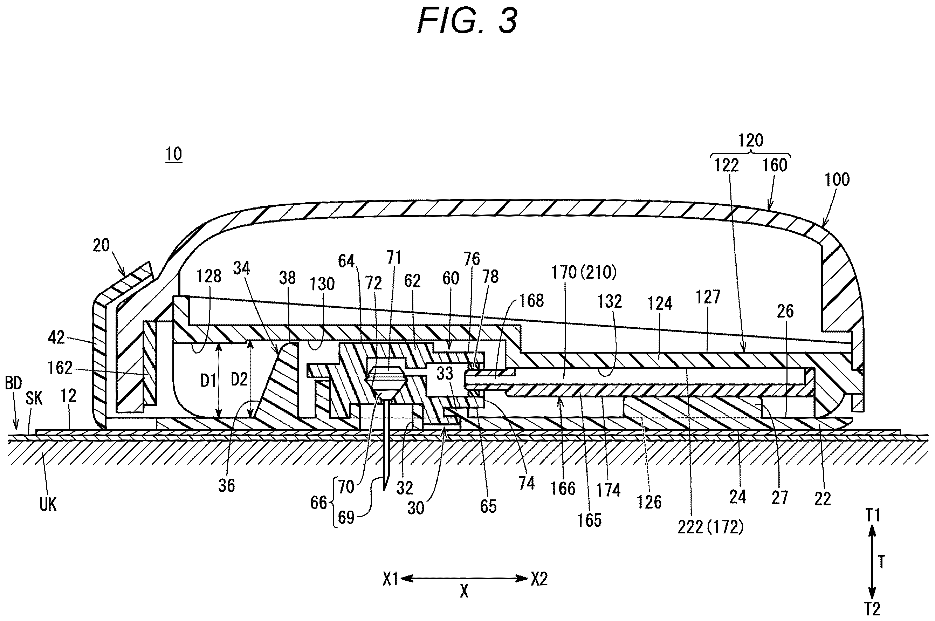

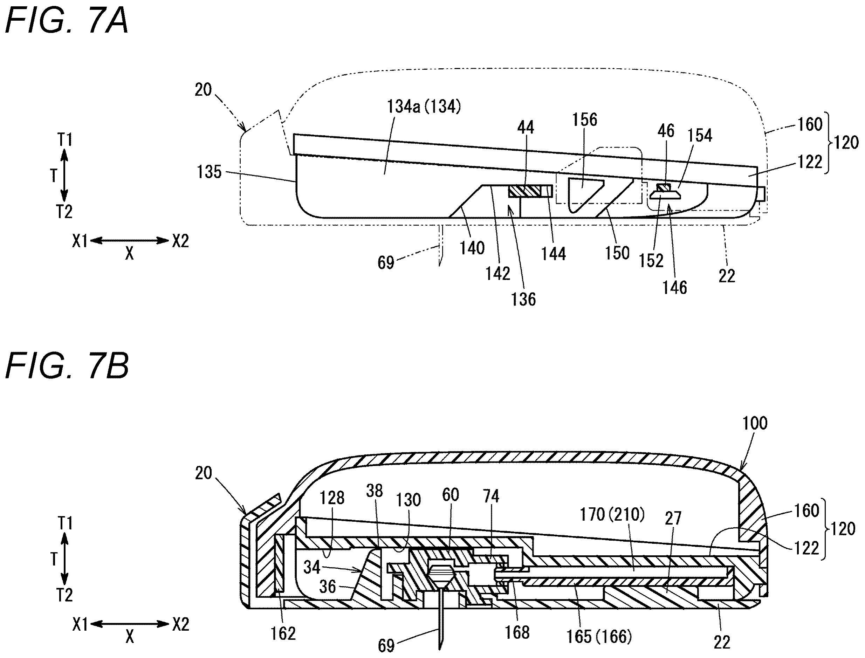

The drug solution administration system 10 includes the holder 20 and a drug solution administration device 100. As understood with reference to Figs. 7A to 10B, the drug solution administration device 100 is detachably mounted to the holder 20. The drug solution administration device 100 moves relative to the holder 20 along a holder inner surface 26 (see Fig. 2) when mounted to or detached from the holder 20. A relative movement direction of the drug solution administration device 100 when the drug solution administration device 100 is mounted to the holder 20 is a direction in which a first connecting portion 74 (see Fig. 3) and a second connecting portion 168 which are to be described later are connected to each other. This direction is hereinafter referred to as connection direction X1. A relative movement direction of the drug solution administration device 100 when the drug solution administration device 100 is detached from the holder 20 is a direction in which the first connecting portion 74 and the second connecting portion 168 are separated from each other, and is opposite to the connection direction X1. This direction is hereinafter referred to as detachment direction X2. A direction along the connection direction X1 and the detachment direction X2 is referred to as axial direction X, and a direction orthogonal to the axial direction X in a direction parallel to the holder inner surface 26 is referred to as width direction W.

As illustrated in Fig. 2, the holder 20 includes a base portion 22. The base portion 22 includes the holder outer surface 24 and the holder inner surface 26. The adhesive sheet 12 is provided on the holder outer surface 24. When the holder 20 is fixed to the skin SK of the patient via the adhesive sheet 12, the holder outer surface 24 faces the skin SK (the body BD). The holder inner surface 26 is the surface (back surface) opposite to the holder outer surface 24.

Hereinafter, the direction extending from the holder outer surface 24 toward the holder inner surface 26 is referred to as thickness direction T. The thickness direction T is orthogonal to both the axial direction X and the width direction W. In the thickness direction T, a direction from the holder outer surface 24 toward the holder inner surface 26 is referred to as upward direction T1. In the thickness direction T, a direction opposite to the upward direction T1 and extending from the holder inner surface 26 toward the holder outer surface 24 is referred to as downward direction T2.

The base portion 22 includes a unit holding portion 30 that protrudes in the thickness direction T (upward direction T1) from the holder inner surface 26. The unit holding portion 30 holds a cannula unit 60, which is described later, on the holder 20. The unit holding portion 30 is formed with a through-hole 32 that penetrates in the thickness direction T of the base portion 22. The through-hole 32 is positioned between the two end portions of the base portion 22 in the axial direction X and between the two end portions of the base portion 22 in the width direction W. A cannula 66 (see Figs. 1 and 3) passes through the through-hole 32. The unit holding portion 30 includes a hook 33. The hook 33 functions as a retaining structure when an unintended force directed in the upward direction T1 acts on the cannula unit 60.

The base portion 22 includes a protection portion 34 that protrudes from the holder inner surface 26 in the thickness direction T. The protection portion 34 is provided on the connection direction X1 side relative to the unit holding portion 30 and extends in the thickness direction T of the base portion 22. A protruding direction of the protection portion 34 is the upward direction T1, which is one direction in the thickness direction T of the base portion 22, and is opposite to the skin SK. In the protection portion 34, an end surface 36 on the connection direction X1 side is inclined toward the detachment direction X2 as the end surface 36 extends from the holder inner surface 26 toward a top portion 38 of the protection portion 34. That is, the end surface 36 is an inclined surface.

The holder 20 includes two holder side portions 40 that are continuous with the base portion 22. The two holder side portions 40 are a first side portion 40a and a second side portion 40b. The first side portion 40a and the second side portion 40b protrude in the upward direction T1, similarly to the protection portion 34. When the drug solution administration device 100 is mounted to the holder 20, the first side portion 40a and the second side portion 40b sandwich a casing 120 in the width direction W. The end portion of the first side portion 40a in the connection direction X1 and the end portion of the second side portion 40b in the connection direction X1 are connected via an arch-shaped portion 42. The first side portion 40a, the second side portion 40b, and the arch-shaped portion 42 integrally form a standing wall portion 43. The standing wall portion 43 is shaped to follow a peripheral portion 135 including a first side surface 134a and a second side surface 134b of the drug solution administration device 100.

In the holder 20 of the illustrated example, a guide rib 44 and a protrusion 46 are provided on the inner surface of the first side portion 40a. The guide rib 44 and the protrusion 46 protrude inward in the width direction W. The first side portion 40a includes a stepped portion 48 provided between the guide rib 44 and the protrusion 46. The stepped portion 48 of the first side portion 40a slightly protrudes outward in the width direction W relative to the outer surface of the first side portion 40a. A movement space 50 is formed inward of the stepped portion 48 of the first side portion 40a. Similarly, the second side portion 40b is also provided with the guide rib 44, the protrusion 46, and the stepped portion 48. The stepped portion 48 of the second side portion 40b also slightly protrudes outward in the width direction W relative to the outer surface of the second side portion 40b. The movement space 50 is formed inward of the stepped portion 48 of the second side portion 40b. The two guide ribs 44 are provided at positions facing each other. Similarly, the two protrusions 46 are provided at positions facing each other. In addition, the two stepped portions 48 are also provided at positions facing each other.

When a drug solution is administered to the patient using the drug solution administration system 10, the drug solution administration device 100 is attached to the holder 20. As illustrated in Fig. 3, the cannula unit 60 is provided in the holder 20. Specifically, the cannula unit 60 includes a housing 62. The housing 62 is attached to the unit holding portion 30 of the holder inner surface 26. The housing 62 attached to the unit holding portion 30 (the holder inner surface 26) protrudes from the holder inner surface 26 in the upward direction T1 (the protruding direction of the protection portion 34), which is one direction in the thickness direction T of the base portion 22. In the illustrated example, regarding the height from the holder inner surface 26 in the upward direction T1, the height of a top portion 64, which is the outer surface of the housing 62 in the upward direction T1, is equal to or smaller than the height of the top portion 38 of the protection portion 34. In other words, the height of the top portion 38 of the protection portion 34 is equal to or greater than the height of the top portion 64 of the housing 62.

The housing 62 includes a locking recess 65. When the user performs a predetermined operation using a puncture device which is not illustrated and is previously mounted to the holder 20, the housing 62 rotates. In conjunction with this rotation, the hook 33 provided on the holder 20 engages with the locking recess 65. As a result, the cannula unit 60 is positioned and fixed to the unit holding portion 30.

The cannula unit 60 includes the cannula 66 provided in the housing 62. As illustrated in Fig. 3, the cannula 66 includes a main body portion 69 positioned at a distal end and a flare portion 70 positioned at a proximal end. A fixing member 71 is mounted to the upper portion of the flare portion 70. The fixing member 71 is fitted into a fitting recess 72 formed in the housing 62. By this fitting, the cannula 66 is held in the housing 62 by the fixing member 71. When the user performs a predetermined operation using a puncture tool, the fixing member 71 holding the cannula 66 approaches the unit holding portion 30, and the main body portion 69 of the cannula 66 is exposed from the through-hole 32 together with a puncture needle of the puncture device. The puncture needle and the main body portion 69 exposed from the through-hole 32 are inserted into the skin SK of the patient.

The housing 62 includes the first connecting portion 74. The first connecting portion 74 protrudes from a location of the housing 62 in the detachment direction X2. The protruding direction of the first connecting portion 74 is the detachment direction X2. A port 76 is formed inside the first connecting portion 74. The port 76 communicates with a lumen (not illustrated) of the cannula 66. A diaphragm 78 formed of rubber or the like is arranged in the port 76. The diaphragm 78 may be, for example, a valve body with a slit. Before the first connecting portion 74 is connected to the second connecting portion 168, the diaphragm 78 is closed. That is, the first connecting portion 74 is sealed by the diaphragm 78. By this sealing, the diaphragm 78 protects the cannula 66 from the external environment.

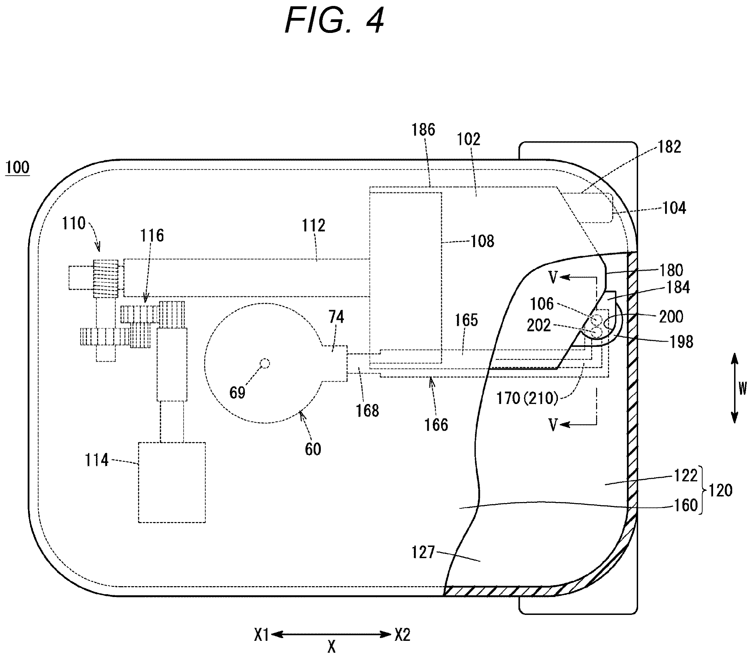

As illustrated in Fig. 4, the drug solution administration device 100 includes a reservoir 102 and the casing 120 that accommodates the reservoir 102. The reservoir 102 extends in the axial direction X of the drug solution administration device 100. Accordingly, the axial direction of the reservoir 102 is parallel to the axial direction X. Therefore, hereinafter, the axial direction of the reservoir 102 may also be referred to as axial direction X.

In the reservoir 102, an end portion (distal end) in the detachment direction X2 includes a closed dome portion 180. The dome portion 180 includes a first connection portion 182 and a second connection portion 184. The first connection portion 182 extends in the detachment direction X2 from one end portion in the width direction W of the dome portion 180. The first connection portion 182 includes a drug solution inlet 104 at a distal end in the detachment direction X2. The drug solution is supplied into the reservoir 102 through the drug solution inlet 104 by the user performing a predetermined operation.

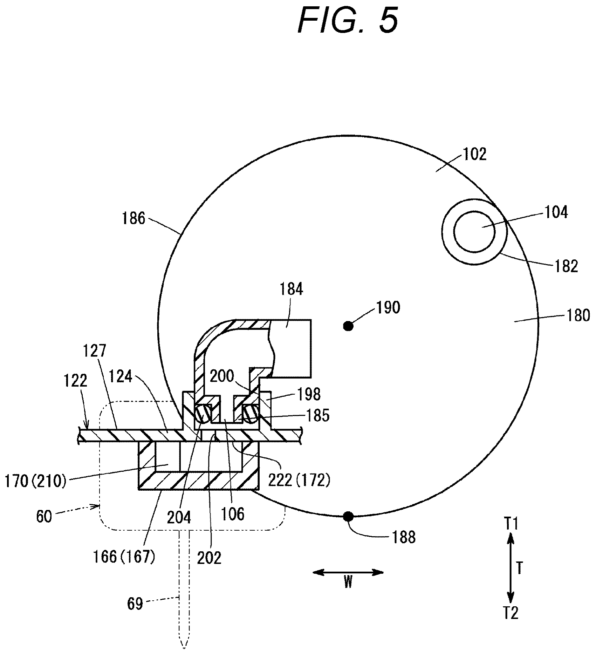

The second connection portion 184 protrudes from the other end portion in the width direction W of the dome portion 180 with respect to an end surface of the dome portion 180. As illustrated in Fig. 5, when the second connection portion 184 is viewed in the detachment direction X2, the second connection portion 184 is positioned inward (on a center side of a cross section in the width direction W of the reservoir 102) relative to a curved side surface 186, which will be described later. The second connection portion 184 forms an L-shaped flow path that is bent from the width direction W toward the downward direction T2. The second connection portion 184 includes a protrusion 185 that protrudes in the downward direction T2 and a drug solution outlet 106 formed in the protrusion 185. An outer diameter of the protrusion 185 is smaller than an outer diameter of the second connection portion 184.

Here, a bottom portion 124 of a first half 122 constituting the casing 120 includes a cylindrical engagement portion 198 on a bottom inner surface 127. The cylindrical engagement portion 198 is formed by an annular wall that extends in the upward direction T1 from the bottom inner surface 127. The cylindrical engagement portion 198 has a connection hole 200 therein. The protrusion 185 of the second connection portion 184 is engaged with the cylindrical engagement portion 198. Accordingly, the drug solution outlet 106 and the connection hole 200 communicate with each other.

A bottom portion inside the cylindrical engagement portion 198 is penetrated by a communication hole 202. As will be described later, the drug solution discharged from the reservoir 102 is supplied to a drug solution flow path 210 via the drug solution outlet 106 and the communication hole 202. An O-ring 204 is mounted on the outer periphery of the protrusion 185. The O-ring 204 liquid-tightly seals between the inner peripheral surface of the connection hole 200 and the outer peripheral surface of the protrusion 185. This prevents the drug solution from leaking from the connection hole 200.

As illustrated in Fig. 5, the reservoir 102 includes the curved side surface 186. The curved side surface 186 curves to form a convex shape toward a flow path forming member 166 that forms the drug solution flow path 210. The reservoir 102 includes a lowermost portion 188 that is positioned at the lowermost location in the downward direction T2. The reservoir 102 also includes a central portion 190 that is positioned at a central location in the upward direction T1, which is one direction in the thickness direction T. In the illustrated example, the shape of the reservoir 102 when viewed in the detachment direction X2 is substantially circular; however, the shape of the reservoir 102 when viewed in the detachment direction X2 may alternatively be a polygonal shape such as a rectangular shape, be an elliptical or oblong shape, etc.

As illustrated in Fig. 4, a gasket 108 moves inside the reservoir 102 in the detachment direction X2, which is one direction in the axial direction X. A pump unit 110 is accommodated inside the casing 120. The pump unit 110 includes a plunger 112 that pushes the gasket 108, and a motor 114 that drives the plunger 112. The motor 114 and the plunger 112 are connected via a plurality of gears 116. When the motor 114 is driven, the plunger 112 advances toward the drug solution outlet 106 positioned on the side in the detachment direction X2. Along with this advancement, the drug solution inside the reservoir 102 is pushed by the gasket 108 and discharged from the drug solution outlet 106.

The casing 120 may be separable into the first half 122 and a second half 160. In that case, for example, among the components of the pump unit 110, the reservoir 102, the plunger 112, a part of the plurality of gears 116, etc. are arranged in the first half 122, while the motor 114, the remaining part of the plurality of gears 116, an electronic board, etc. are arranged in the second half 160. Further, a battery (not illustrated) for driving the gears 116 is arranged in either the first half 122 or the second half 160.

As illustrated in Fig. 3, the casing 120 includes a first half 122 and a second half 160. The first half 122 includes a bottom portion 124. The bottom portion 124 includes a base side portion 134, a bottom outer surface 126, and the bottom inner surface 127, which will be described later. The bottom outer surface 126 is an outer surface facing the holder 20, and the bottom inner surface 127 is a back surface of the bottom outer surface 126. By erecting the base side portion 134 in the thickness direction T from the peripheral portions of the bottom outer surface 126 and the bottom inner surface 127, components constituting the pump unit 110 can be accommodated inside the first half 122. When the reservoir 102 and the bottom inner surface 127 are taken as a reference, the downward direction T2 can be described as a direction from the reservoir 102 and the bottom inner surface 127 toward the bottom outer surface 126. The upward direction T1 can be described as a direction from the bottom outer surface 126 toward the bottom inner surface 127 and the reservoir 102 (a direction away from the bottom outer surface 126).

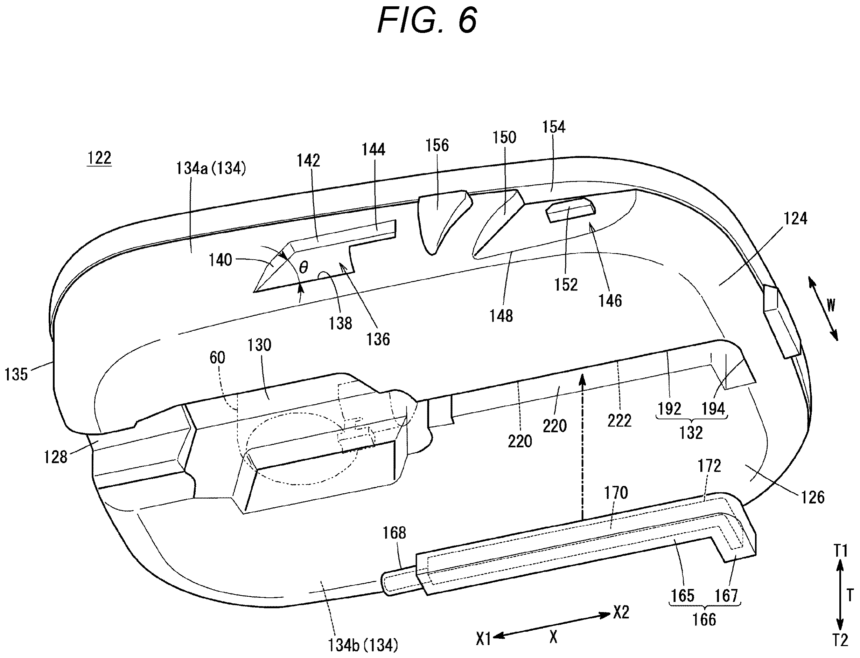

When the drug solution administration device 100 is mounted to the holder 20, the bottom outer surface 126 of the bottom portion 124 faces the holder inner surface 26 of the base portion 22 of the holder 20. As illustrated in Figs. 3 and 6, the bottom outer surface 126 of the bottom portion 124 includes a semi-circular insertion recess 128, an accommodation recess 130, and a recessed groove 132. The insertion recess 128, the accommodation recess 130, and the recessed groove 132 are arranged in this order from the connection direction X1 toward the detachment direction X2 and are recessed in a direction (the upward direction T1) away from the holder inner surface 26 of the holder 20. The insertion recess 128, the accommodation recess 130, and the recessed groove 132 form a continuous space.

As illustrated in Fig. 3, an opening 172 at the end portion of the insertion recess 128 in the connection direction X1 is covered with an end wall portion 162 of the second half 160. As a result, the insertion recess 128 opens only at a portion facing the holder inner surface 26. As understood from the foregoing, the end wall portion 162 is the end portion of the insertion recess 128 in the connection direction X1. The end wall portion 162 faces the arch-shaped portion 42 of the holder 20 when the drug solution administration device 100 is mounted to the holder 20. In a state in which the drug solution administration device 100 is mounted to the holder 20, the protection portion 34 is positioned between the end wall portion 162 and the housing 62 of the cannula unit 60, and is positioned within the accommodation recess 130. When the drug solution administration device 100 is removed from the holder 20, the protection portion 34 first relatively moves from the accommodation recess 130 into the insertion recess 128.

In a state in which the drug solution administration device 100 is mounted to the holder 20, the cannula unit 60 is accommodated in the accommodation recess 130. A distance (depth D2) from the bottom outer surface 126 of the bottom portion 124 to a ceiling surface of the accommodation recess 130 is longer than a distance (depth D1) from the bottom outer surface 126 of the bottom portion 124 to a ceiling surface of the insertion recess 128.

As illustrated in Fig. 6, the recessed groove 132 is substantially L-shaped. Specifically, the recessed groove 132 includes a first straight groove 192 that extends linearly in an axial direction (axial direction X) of the reservoir 102, and a second straight groove 194 that extends linearly to some extent in the width direction W. The second straight groove 194 is connected to an end portion of the first straight groove 192 on the detachment direction X2 side such that the second straight groove 194 is bent approximately 90 degrees toward a longitudinal central axis of the reservoir 102. The second straight groove 194 is shorter than the first straight groove 192. Thus, when the first half 122 is viewed in a direction from the downward direction T2 toward the upward direction T1, the recessed groove 132 is substantially L-shaped.

The recessed groove 132 includes two groove inner side surfaces 220 and a ceiling surface 222 (see Fig. 3). The two groove inner side surfaces 220 and the ceiling surface 222 are the inner surfaces of the recessed groove 132 and are also a part of the bottom outer surface 126. The ceiling surface 222 is a ceiling surface of the recessed groove 132. The two groove inner side surfaces 220 face each other across the ceiling surface 222.

The drug solution administration device 100 includes the flow path forming member 166, as illustrated in Figs. 3 to 6. When the first half 122 is viewed in a direction from the downward direction T2 toward the upward direction T1, the flow path forming member 166 is substantially L-shaped corresponding to the shape of the recessed groove 132 (see Fig. 6). Specifically, the flow path forming member 166 includes a first straight portion 165 that extends linearly in an axial direction (axial direction X) of the reservoir 102, and a second straight portion 167 that extends linearly to some extent along the width direction W. The second straight portion 167 is connected to an end portion of the first straight portion 165 on the detachment direction X2 side such that the second straight portion 167 is bent approximately 90 degrees toward the reservoir 102. The second straight portion 167 is shorter than the first straight portion 165.

The flow path forming member 166 includes a hollow interior 170 and the opening 172 (see especially Fig. 5). The opening 172 is formed in a side portion of the flow path forming member 166 that faces the ceiling surface 222 of the recessed groove 132. The hollow interior 170 communicates with the opening 172. Therefore, when the flow path forming member 166 is cut in the width direction W and viewed in the axial direction X, its cross section is substantially U-shaped. The opening 172 opens in the upward direction T1. The hollow interior 170 is bent in a substantially L-shaped manner corresponding to the shape of the flow path forming member 166 (see Fig. 6). The flow path forming member 166 further includes the second connecting portion 168 at the end portion in the connection direction X1. The opening 172 is not formed in the second connecting portion 168. That is, the second connecting portion 168 is a tubular portion having a lumen. The end surface of the second connecting portion 168 in the connection direction X1 may be flat.

In the present embodiment, the material of the flow path forming member 166 is resin. The flow path forming member 166 is transparent, allowing a drug solution flowing through the hollow interior 170 (the drug solution flow path 210) to be visually observed from outside the flow path forming member 166. Examples of such resin include polypropylene, polycarbonate, polyethylene, and acrylonitrile-butadiene-styrene copolymer. Here, the material of the flow path forming member 166 is not limited to resin. The material of the flow path forming member 166 may also be metal.

As illustrated in Fig. 3, the flow path forming member 166 is accommodated in the recessed groove 132. Specifically, the first straight portion 165 is accommodated in the first straight groove 192, and the second straight portion 167 is accommodated in the second straight groove 194 (see Fig. 6). The length of the flow path forming member 166 in the thickness direction T is shorter than the length (depth) of the recessed groove 132 in the thickness direction T. Therefore, in the thickness direction T, the entire flow path forming member 166 fits within the recessed groove 132 (see Fig. 3). In other words, in the thickness direction T, the flow path forming member 166 is not exposed from the recessed groove 132. As a result, a guide recess 174 is formed between a surface positioned at the lowermost position in the downward direction T2 on the bottom outer surface 126 and a lower surface of the first straight portion 165. The guide recess 174 extends in the axial direction X. In contrast, the second connecting portion 168 is exposed from the recessed groove 132 in the connection direction X1 and is positioned within the accommodation recess 130.

The end surface of the flow path forming member 166 in the upward direction T1 comes into contact with the ceiling surface 222 of the recessed groove 132, for example, as illustrated in Fig. 3. The end surface and the ceiling surface 222 are joined by, for example, ultrasonic welding or adhesive, with ultrasonic welding being most preferred. As a result, the opening 172 of the flow path forming member 166 is closed by the ceiling surface 222 (i.e., the bottom outer surface 126). Alternatively, a sealing material may be inserted between the end surface of the flow path forming member 166 and the ceiling surface 222.

As described above, the recessed groove 132, which is a part of the bottom outer surface 126, and the hollow interior 170 together form the drug solution flow path 210. Note that it is also possible to form the drug solution flow path 210 without causing the end surface of the flow path forming member 166 in the upward direction T1 to contact the ceiling surface 222 of the recessed groove 132. The end surface of the flow path forming member 166 in the upward direction T1 may be separated from the ceiling surface 222 of the recessed groove 132. For example, the flow path forming member 166 may be joined to the two groove inner side surfaces 220, or the flow path forming member 166 may be joined to the bottom outer surface 126 in a manner that the flow path forming member 166 covers the entire recessed groove 132. In these cases, the recessed groove 132 and the hollow interior 170 form the drug solution flow path 210 by communicating with each other via the opening 172. Here, it is preferable to form the drug solution flow path 210 by joining the flow path forming member 166 in a manner that the entire flow path forming member 166 fits within the recessed groove 132 in order to reduce dead volume within the drug solution flow path 210.

As illustrated in Figs. 4 and 5, when viewed in the downward direction T2, at least a part of the drug solution flow path 210 overlaps with the position of the reservoir 102. That is, on the downward direction T2 side of the syringe-type reservoir 102, the drug solution flow path 210 is arranged along a gap that occurs in the longitudinal direction of the reservoir 102. The first straight portion 165 of the flow path forming member 166 and the drug solution flow path 210 are positioned on the downward direction T2 side across the first half 122 in the width direction W of the reservoir 102. Here, the length of the flow path forming member 166 in the axial direction X is slightly longer than the length of the reservoir 102 in the axial direction X (see Fig. 4). Therefore, the first straight portion 165 and the drug solution flow path 210 overlap with one end portion in the width direction W of the reservoir 102 over a range extending from the end portion on the connection direction X1 side of the dome portion 180 of the reservoir 102 to the end portion on the detachment direction X2 side of the reservoir 102.

Further, as illustrated in Fig. 5, when the drug solution administration device 100 is viewed in the detachment direction X2, the drug solution flow path 210 is positioned, in the thickness direction T, between the lowermost portion 188 of the reservoir 102 and the central portion 190 of the reservoir 102. Here, the lowermost portion 188 refers to a portion of the reservoir 102 that is positioned at the lowest position (on the downward direction T2 side). Therefore, when the drug solution administration device 100 is viewed in the width direction W, the first straight portion 165 and the drug solution flow path 210 are positioned between the lowermost portion 188 of the reservoir 102 and the central portion 190 of the reservoir 102. In other words, in this case, the first straight portion 165 and the drug solution flow path 210 overlap a portion between the lowermost portion 188 of the reservoir 102 and the central portion 190 of the reservoir 102.

In a case where a drug solution flow path is provided by arranging a tubular member along a gap that occurs in the longitudinal direction of the reservoir 102 on the downward direction T2 side of the syringe-type reservoir 102, it is necessary to assemble the tubular member by pushing the tubular member into the gap between the reservoir 102 and the bottom inner surface 127. This results in a complicated manufacturing process of the drug solution administration device 100. Moreover, it is difficult to automate the assembly of the drug solution administration device 100. In contrast, by using the flow path forming member 166 as a resin member and adopting the configuration described above, the manufacturing process can be simplified. In addition, automation of the assembly of the drug solution administration device 100 is facilitated.

As illustrated in Fig. 3, the second connecting portion 168 is connected to the first connecting portion 74. As a result, the drug solution administration device 100 is mounted to the holder 20. Meanwhile, as illustrated in Fig. 5, the drug solution outlet 106 of the reservoir 102 and the hollow interior 170 of the flow path forming member 166 communicate with each other via the connection hole 200 and the communication hole 202. Accordingly, the drug solution outlet 106 of the reservoir 102 and the port 76 of the housing 62 are connected to each other via the drug solution flow path 210.

As illustrated in Fig. 6, the first half 122 includes the base side portion 134. The base side portion 134 includes the first side surface 134a and the second side surface 134b. The first side surface 134a faces the inner surface of the first side portion 40a of the holder 20. The second side surface 134b faces the inner surface of the second side portion 40b of the holder 20.

A guide groove 136 is formed in the first side surface 134a. The guide groove 136 is positioned substantially at the center in the axial direction X on the first side surface 134a. The guide groove 136 is continuous with a first insertion and removal opening 138. The first insertion and removal opening 138 is formed at the boundary between the bottom portion 124 and the first side surface 134a of the first half 122 and extends in the axial direction X. When the first half 122 is viewed from the bottom outer surface 126 side, the first insertion and removal opening 138 is formed as a notch recessed inward in the width direction W on the first side surface 134a. The first insertion and removal opening 138 opens a portion of the first side surface 134a toward the bottom outer surface 126 side.

The guide groove 136 includes a first inclined portion 140 that is continuous with the first insertion and removal opening 138. The first inclined portion 140 is recessed inward in the width direction W from the outer surface of the first side surface 134a, and is positioned on the connection direction X1 side among the side surfaces of the guide groove 136 surrounding the recess. When the guide groove 136 is viewed from the first side surface 134a, the first inclined portion 140 inclines in a direction away from the base portion 22 (that is, the bottom outer surface 126 of the bottom portion 124) with respect to the axial direction X as the first inclined portion 140 extends from the connection direction X1 toward the detachment direction X2. An intersection angle θ between the first inclined portion 140 and the axial direction X is, for example, 40° to 50°, and typically 45°. Furthermore, the guide groove 136 includes a horizontal groove portion 142 that extends in the axial direction X and connects, on the thickness direction T side of the first insertion and removal opening 138, from the first inclined portion 140 toward the detachment direction X2. The horizontal groove portion 142 is a surface recessed inward in the width direction W from the outer surface of the first side surface 134a, and is a portion positioned on the upward direction T1 side among the side surfaces surrounding the guide groove 136.

At the end portion of the horizontal groove portion 142 in the detachment direction X2, an engagement recess 144 is continuous therewith. The engagement recess 144 is a recess having a rectangular shape that is narrower in width than the guide groove 136, and extends in the axial direction X. The guide groove 136 and the engagement recess 144 are continuous with each other, thereby forming one recess. As will be described later, the guide rib 44 is inserted into the guide groove 136 and the engagement recess 144 in a movable manner in the X direction (see Figs. 7A, 8A, 9A, and 10A).

On the first side surface 134a, at a position on a side in the detachment direction X2 relative to the guide groove 136, a recess 146 is formed. The recess 146 is provided for insertion (clearance) of the protrusion 46. The recess 146 is formed by recessing the side surface of the first half 122. This prevents interference of the protrusion 46 with the first side surface 134a. A second insertion and removal opening 148 is continuous with the recess 146. The second insertion and removal opening 148 is formed at the boundary between the bottom outer surface 126 and the first side surface 134a of the first half 122 and extends in the axial direction X. When the first half 122 is viewed from the bottom outer surface 126 side, the second insertion and removal opening 148 is formed as a notch recessed inward in the width direction W on the first side surface 134a. The second insertion and removal opening 148 opens a portion of the first side surface 134a toward the bottom outer surface 126 side.

The recess 146 includes a second inclined portion 150 that is substantially parallel to the first inclined portion 140 of the guide groove 136. The protrusion 46 is inserted into the recess 146 in a movable manner. An engagement portion 152 is provided inside the recess 146. The engagement portion 152 bulges outward in the width direction W of the first half 122. As illustrated in Figs. 7A, 8A, 9A, and 10A, a lock groove 154 is formed between the upper surface of the engagement portion 152 and the ceiling surface of the recess 146.

Between the guide groove 136 and the recess 146 on the first side surface 134a, a bulging portion 156 is provided. The bulging portion 156 bulges outward in the width direction W of the first half 122. The bulging portion 156 has a substantially right triangular shape.

Similarly to the first side surface 134a, the second side surface 134b is also provided with the guide groove 136, the recess 146, and the bulging portion 156.

Instead of forming the guide groove 136 on each of the first side surface 134a and the second side surface 134b, the guide rib 44 may be provided on each of the first side surface 134a and the second side surface 134b. In this case, the guide groove 136 is formed on each of the inner surface of the first side portion 40a and the inner surface of the second side portion 40b of the holder 20.

In the case where the guide rib 44 is provided on the holder 20, the position of the guide rib 44 is not limited to the first side portion 40a and the second side portion 40b. For example, the guide rib 44 may be provided on the holder inner surface 26 in the vicinity of the end portion facing the detachment direction X2. In this case, the guide groove 136 is formed to be recessed toward the second half 160 in the vicinity of the end portion facing the detachment direction X2 on the bottom outer surface 126 of the bottom portion 124 of the first half 122, for example. In this configuration, for example, it is possible to provide the first inclined portion 140, whose height increases toward the detachment direction X2, on the guide rib 44 while keeping the depth of the guide groove 136 constant. Alternatively, the guide rib 44 may be formed into a rectangular column shape without the first inclined portion 140, while the guide groove 136 may be provided with the first inclined portion 140 whose height increases toward the detachment direction X2. Furthermore, the first inclined portion 140 may be provided on both the guide rib 44 and the guide groove 136.

On the second side surface 134b, constituent elements having the same configuration as described above are provided at positions mirror-symmetrical to the first side surface 134a with respect to the axial direction X. That is, on the second side surface 134b, the first inclined portion 140, the guide groove 136, the first insertion and removal opening 138, the horizontal groove portion 142, the bulging portion 156, the second insertion and removal opening 148, the engagement portion 152, the recess 146, and the second inclined portion 150 are provided. Further, on the holder 20, the guide rib 44, the protrusion 46, and the like are provided at positions corresponding to each of these constituent elements of the second side surface 134b. Thus, by arranging constituent elements that serve the same functions at positions symmetrical to each other with respect to the axial direction X on the first side surface 134a and the second side surface 134b, the attachment and detachment operations of the drug solution administration device 100 to and from the holder 20 can be performed stably.

A ridge guide 27 is provided on the holder inner surface 26. The ridge guide 27 is a protrusion in the form of a ridge that extends in the axial direction X, and guides movement of the drug solution administration device 100 in the axial direction X when the drug solution administration device 100 is mounted to or detached from the holder 20. In a state in which the holder 20 is mounted to the drug solution administration device 100, the ridge guide 27 corresponds to the position of the recessed groove 132. Accordingly, when the drug solution administration device 100 is mounted to or detached from the holder 20, an upper portion of the ridge guide 27 is inserted into the guide recess 174. Therefore, when the drug solution administration device 100 moves in the axial direction X, the drug solution administration device 100 is guided by the ridge guide 27. As a result, during attachment and detachment operations of the drug solution administration device 100, the user can stably slide the drug solution administration device 100 on the holder 20.

Thus, by accommodating the entirety of the first straight portion 165 of the flow path forming member 166 in the first straight groove 192, and forming the guide recess 174 recessed in the upward direction T1 between the lowermost portion of the bottom outer surface 126 and the lower surface of the first straight portion 165, a guide mechanism can be provided by the ridge guide 27 and the guide recess 174. Therefore, it is unnecessary to provide a separate guide groove on the casing 120.

The second half 160 is connected to the first half 122 to cover the entire upper part of the first half 122. As a result, the reservoir 102 and the pump unit 110 are accommodated in the hollow interior 170 formed by the second half 160 and the first half 122. As described above, the end wall portion 162 of the second half 160 constitutes the end portion of the insertion recess 128 in the connection direction X1.

The drug solution administration system 10 is used as follows. First, the user fills the reservoir 102 inside the first half 122 with a drug solution via the drug solution inlet 104 using, for example, a predetermined drug solution filling device.

Next, the user pushes the plunger 112 toward a distal end of the reservoir 102 using a coin or the like, with the drug solution outlet 106 of the reservoir 102 facing upward. As a result, as the plunger 112 moves in the detachment direction X2, most of the air in the reservoir 102 is pushed out by the gasket 108. As can be understood from this, the pushing direction on the drug solution in the reservoir 102 is the detachment direction X2. Thereafter, the user attaches the second half 160 to the first half 122.

Next, the user operates a remote controller, which is not illustrated, to perform a priming operation to fill the drug solution flow path 210 with the drug solution. For this purpose, the motor 114 is driven. As a result, the drug solution in the reservoir 102, which is pushed by the gasket 108, and air that still remains in the drug solution flow path 210 and the like, are discharged from the drug solution outlet 106. The drug solution moves into the connection hole 200, and then into the hollow interior 170 of the flow path forming member 166 via the communication hole 202 and the opening 172. In this way, the drug solution flows into the drug solution flow path 210. Since the flow path forming member 166 is substantially L-shaped and the first straight portion 165 extends in the connection direction X1, the flow direction of the drug solution in the drug solution flow path 210 is changed from the width direction W to the connection direction X1. Thus, by configuring the flow path forming member 166 to be substantially L-shaped, it is easy to change the direction of the drug solution, which has been pushed out from the reservoir 102 in the detachment direction X2, to the connection direction X1, which is opposite to the detachment direction X2.

Thereafter, the drug solution moves along the drug solution flow path 210 in the connection direction X1. A small amount of the drug solution that has reached the second connecting portion 168 is discharged from a port of the second connecting portion 168. As a result, the user can recognize, in a stage prior to the start of drug solution flow, that the air existing from the interior of the second connection portion 184 to the port of the second connecting portion 168 has been pushed out by the drug solution, and that the drug solution flow path 210 has been filled with the drug solution. In that case, the user terminates the pushing on the drug solution by the plunger 112.

When the flow path forming member 166 is transparent, the user can visually observe the drug solution flow path 210 from outside the flow path forming member 166. Accordingly, the user can promptly confirm, for example, whether any foreign matter is mixed in the drug solution within the drug solution flow path 210 by visual inspection. Alternatively, the user can promptly confirm whether the drug solution within the drug solution flow path 210 has entrained air (whether bubbles are present) by visual inspection.

When administration of the drug solution to the patient is started with a large volume of bubbles remaining in the drug solution flow path 210, the drug solution is not administered to the patient while the bubbles are being discharged from the drug solution flow path 210. Therefore, for example, when a drug solution having a very small prescribed dose (such as insulin) is to be administered to a patient, it is not easy to administer the prescribed dose of the drug solution to the user within a predetermined time period set in the remote controller. In contrast, when the flow path forming member 166 is made of a transparent resin, the user can notice that bubbles remain in the drug solution flow path 210, as described above. Accordingly, the user can operate to reliably discharge the bubbles from the flow path forming member 166 before starting drug solution administration. As a result, even in cases where a drug solution having a very small prescribed dose is administered to the patient, the prescribed dose of the drug solution can be reliably administered to the patient.

Meanwhile, as illustrated in Fig. 3, the user fixes the holder 20 to the skin SK of a predetermined site of the patient via the adhesive sheet 12. An abdomen is exemplified as the predetermined site. At this time point, the cannula unit 60 is not yet held in the unit holding portion 30 of the holder 20. Next, the user performs a predetermined operation using a puncture device which is not illustrated. As a result, the puncture needle of the puncture device and the main body portion 69 of the cannula 66 pass through the through-hole 32 and are inserted into the skin SK of the patient. By the user rotating the housing 62 together with the puncture device, the cannula unit 60 approaches the unit holding portion 30, and the hook 33 of the holder 20 engages with the locking recess 65 of the housing 62.

Accordingly, the cannula unit 60 is positioned and fixed (locked) to the holder 20. Based on this, the state in which the main body portion 69 of the cannula 66 is inserted into a subcutaneous tissue UK of the patient is maintained. That is, the main body portion 69 of the cannula 66 is indwelled in the subcutaneous tissue UK of the patient. Thereafter, the user removes the puncture device from the holder 20. As a result of this operation, only the puncture needle is removed from the subcutaneous tissue UK. Accordingly, only the cannula 66 remains indwelled in the subcutaneous tissue UK.

Next, after performing the above-described visual inspection, the user mounts the drug solution administration device 100 to the holder 20. The mounting procedure is the reverse of the detachment procedure for the drug solution administration device 100 from the holder 20. Since the detachment will be described later, the mounting procedure will be outlined only briefly.

### [First step]

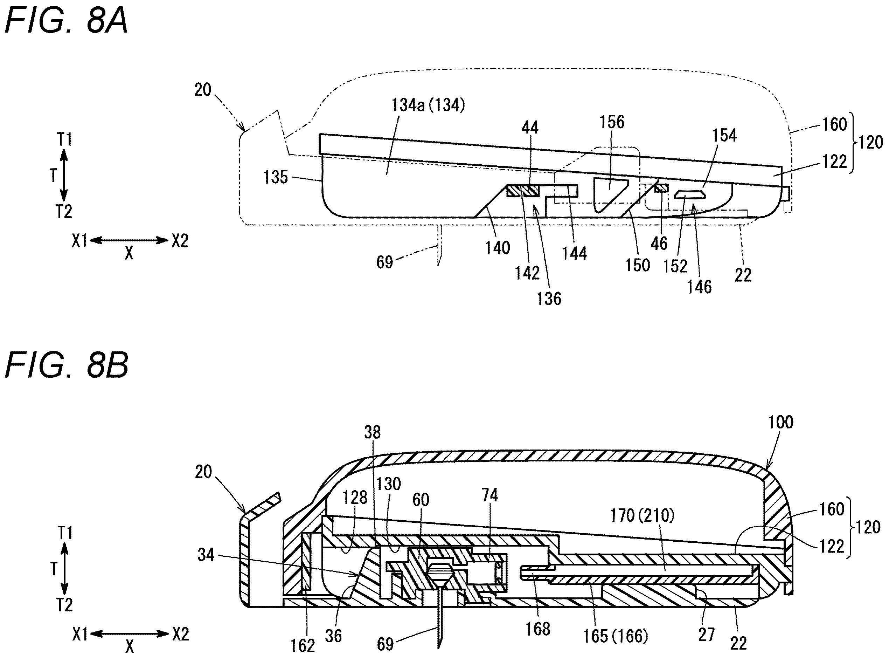

In the process of mounting the drug solution administration device 100 to the holder 20, the user brings the bottom outer surface 126 of the bottom portion 124 of the first half 122 into contact with the holder inner surface 26, as illustrated in Fig. 8A. The user arranges the drug solution administration device 100 toward the base portion 22 (in the thickness direction T) at a position where the protection portion 34 and the cannula unit 60 fit within the accommodation recess 130. At this time, in a side view from the width direction W, a portion of an end side in the detachment direction X2 of the drug solution administration device 100 protrudes in the detachment direction X2 from the upper surface of the holder 20, while another portion of the drug solution administration device 100 is placed on the holder 20.

At this time, the guide rib 44 is positioned at the end portion in the connection direction X1 of the first inclined portion 140 (or the horizontal groove portion 142) of the guide groove 136. At this time point, the first connecting portion 74 of the housing 62 and the second connecting portion 168 of the drug solution administration device 100 are not yet connected. Therefore, the drug solution administration device 100 is not yet mounted to the holder 20. In addition, the protrusion 46 is positioned at the end portion in the connection direction X1 of the second inclined portion 150 of the recess 146.

### [Second step]

From this state, the user slides the drug solution administration device 100 in the connection direction X1 on the holder 20. The drug solution administration device 100 moves relative to the holder 20 in the connection direction X1 along the holder inner surface 26. As a result, the first connecting portion 74 of the housing 62 and the second connecting portion 168 of the flow path forming member 166 are connected to each other (see Fig. 3 and Fig. 7B). Accordingly, the drug solution outlet 106 of the reservoir 102 and the port 76 of the housing 62 are connected via the drug solution flow path 210 (the flow path forming member 166), and the drug solution administration device 100 is mounted to the holder 20.

Along with the above relative movement, the guide rib 44 moves relatively in the detachment direction X2 along the horizontal groove portion 142 of the guide groove 136. As illustrated in Fig. 7A, the guide rib 44 engages with the engagement recess 144 that is continuous with the horizontal groove portion 142 in the detachment direction X2. Similarly, the protrusion 46 moves relative to the recess 146 in the detachment direction X2 and enters between the upper surface of the engagement portion 152 and the ceiling surface of the recess 146. In other words, the protrusion 46 engages with the lock groove 154. Based on the engagement of the guide rib 44 with the engagement recess 144 and the engagement of the protrusion 46 with the lock groove 154, detachment of the drug solution administration device 100 from the holder 20 is prevented.

The protection portion 34 is positioned in the accommodation recess 130 in the state illustrated in Fig. 7A (see Fig. 7B), and positioned in the insertion recess 128 in the state illustrated in Fig. 8A (see Fig. 8B). The bulging portion 156 moves relatively within the movement space 50 (see Fig. 2), and comes into contact with one end of the movement space 50 in the connection direction X1 at a position where the mounting of the drug solution administration device 100 to the holder 20 is completed.

Next, the user operates the remote controller. As a result, the motor 114 (see Fig. 4) is driven, and the plunger 112 and the gasket 108 move in the detachment direction X2. Consequently, the drug solution in the reservoir 102 is pushed by the gasket 108 and discharged from the drug solution outlet 106. The drug solution moves along the drug solution flow path 210 in the connection direction X1, as described above. Next, the drug solution flows from the second connecting portion 168 toward the first connecting portion 74, and subsequently flows through the port 76 of the housing 62 and into the lumen of the cannula 66. The drug solution further flows out from the drug solution administration opening of the lumen. As a result, the drug solution is continuously administered to the patient. Therefore, even while the drug solution is being administered to the patient, the pushing direction on the drug solution in the reservoir 102 is the detachment direction X2, and the flow direction of the drug solution in the drug solution flow path 210 is the connection direction X1, which is opposite to the detachment direction X2.

When administration of the drug solution has been completed, the user removes the drug solution administration device 100 from the holder 20. First, the user moves the drug solution administration device 100 in the detachment direction X2 from the state illustrated in Fig. 7A. The drug solution administration device 100 moves relative to the holder 20 in the detachment direction X2 along the holder inner surface 26, reaching the state illustrated in Fig. 8A. Accordingly, as illustrated in Fig. 8B, the first connecting portion 74 of the housing 62 and the second connecting portion 168 of the flow path forming member 166 are detached from each other. As a result, the drug solution administration device 100 is attached to or detached from the holder 20. As a result, in the side view from the width direction W, the portion of the end side in the detachment direction X2 of the drug solution administration device 100 protrudes in the detachment direction X2 from the upper surface of the holder 20, and another portion of the drug solution administration device 100 is placed on the holder 20.

Along with the partial sliding movement described above, the guide rib 44 is detached from the engagement recess 144, then moves relatively in the connection direction X1 along the horizontal groove portion 142 of the guide groove 136, and reaches the end portion in the connection direction X1 of the horizontal groove portion 142 (or the first inclined portion 140), as illustrated in Fig. 8A. Similarly, the protrusion 46 is detached from the lock groove 154, then moves relatively in the connection direction X1 within the recess 146, and reaches the end portion in the connection direction X1 of the second inclined portion 150. As a result, the drug solution administration device 100 is released from the constraint of the holder 20. The protection portion 34 moves relatively from the accommodation recess 130 to the insertion recess 128.

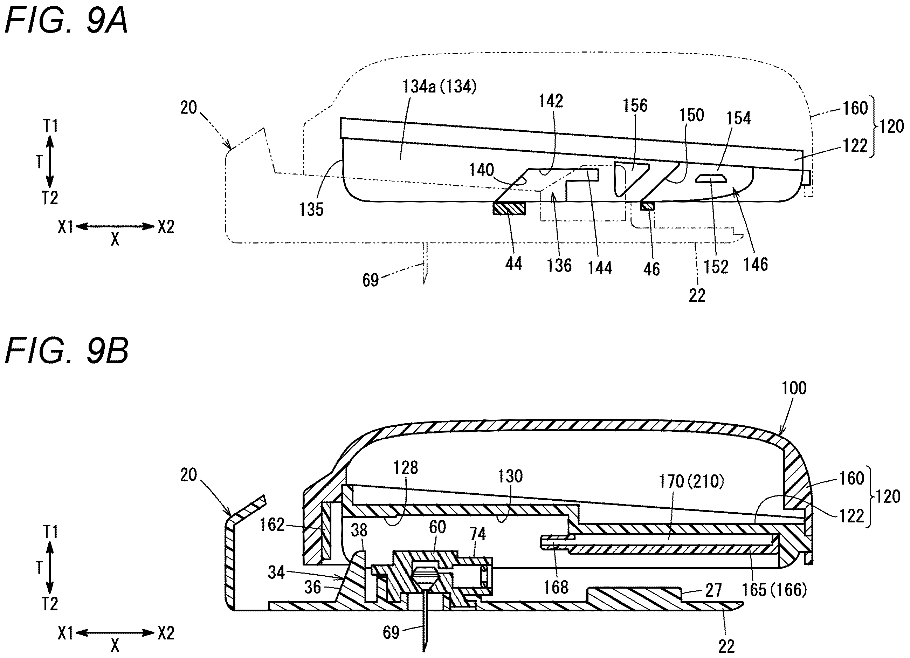

Next, the user moves the drug solution administration device 100 in a direction away from the body BD of the patient. At this time, the guide rib 44 moves relatively while sliding in contact with the inner surface of the first inclined portion 140. Accordingly, as illustrated in Fig. 9A, the drug solution administration device 100 is guided in an inclined direction along the first inclined portion 140 by the guide rib 44 and the first inclined portion 140. That is, the drug solution administration device 100 moves obliquely away from the holder 20, in a direction slanting from the thickness direction T toward the detachment direction X2. In this mode, when the drug solution administration device 100 moves obliquely, the protrusion 46 moves relatively while sliding in contact with the inner surface of the second inclined portion 150. Here, the protrusion 46 may also be separated from the inner surface of the second inclined portion 150. In such a case, the protrusion 46 does not slide in contact with the inner surface of the second inclined portion 150.

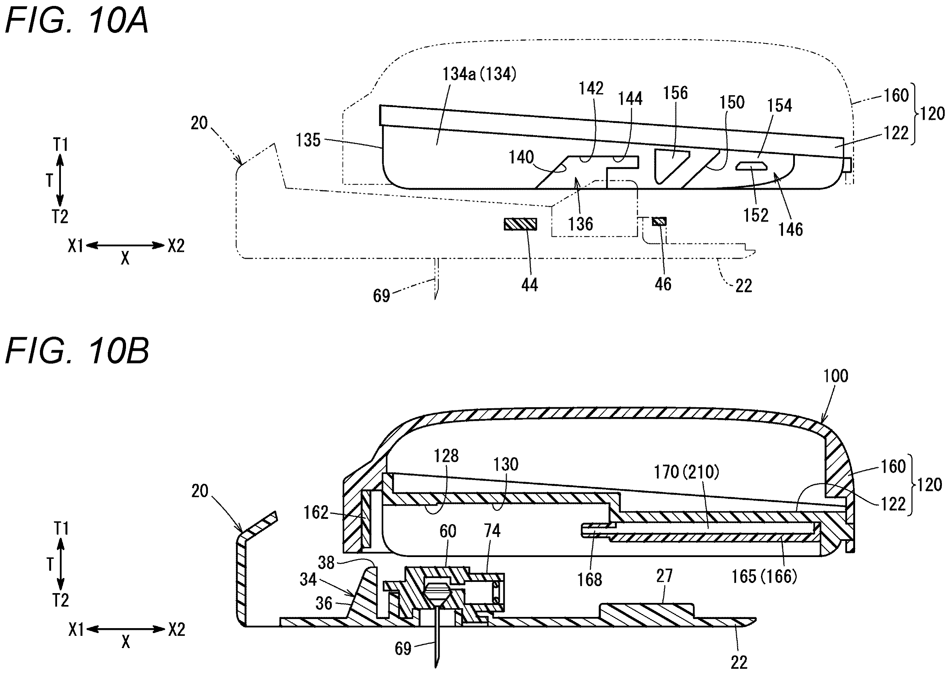

As illustrated in Fig. 9B, at the time point when the guide rib 44 reaches the first insertion and removal opening 138, the top portion 64 of the housing 62 is not exposed from the insertion recess 128. In this state, when the user moves the drug solution administration device 100 in the detachment direction X2, the end wall portion 162 interferes with the top portion 38 of the protection portion 34. Thereafter, the drug solution administration device 100 is guided in an inclined direction along the inclination of the end surface 36 by the end surface 36 in the connection direction X1 of the protection portion 34. Accordingly, the drug solution administration device 100 continues to move obliquely while further separating from the holder 20, as illustrated in Fig. 10A. As a result, interference of the casing 120 with the cannula unit 60 during detachment of the drug solution administration device 100 from the holder 20 is prevented. As illustrated in Fig. 10B, the guide rib 44 is detached from the guide groove 136 via the first insertion and removal opening 138, and the protrusion 46 is detached from the recess 146 via the second insertion and removal opening 148.

As described above, in the side view from the width direction W, by sliding the drug solution administration device 100 on the holder 20 from a position where the portion of the end side in the detachment direction X2 of the drug solution administration device 100 protrudes in the detachment direction X2 from the upper surface of the holder 20 to connect the drug solution flow path 210, the sliding distance of the drug solution administration device 100 is shortened. Thus, by partially sliding the drug solution administration device 100 relative to the holder 20 for connection, the stroke required for the sliding operation is reduced. Therefore, the operation of connecting the drug solution administration device 100 to the holder 20 is facilitated.

Further, when the drug solution administration device 100 is detached from the holder 20, the protection portion 34 can prevent interference of the cannula unit 60 with the casing 120. Therefore, even when the drug solution administration system 10 is mounted to a site that is difficult for the patient to visually confirm, such as the back or waist, the drug solution administration device 100 can still be reliably and easily detached from the holder 20.

The present embodiment has the following effects.

The drug solution administration device 100 includes the reservoir 102 illustrated in Fig. 4 and the casing 120 that accommodates the reservoir 102 (see Figs. 1 and 3). The reservoir 102 is filled with the drug solution to be supplied to the cannula 66 of the cannula unit 60 illustrated in Figs. 1 and 3. The casing 120 has the bottom portion 124 including: the bottom outer surface 126 that faces the living subject when the drug solution is administered to the living subject; and the bottom inner surface 127 that is the back surface of the bottom outer surface 126. As illustrated in Fig. 5, the reservoir 102 is arranged on the bottom inner surface 127. As illustrated in Fig. 6, the bottom outer surface 126 has the recessed groove 132 that is recessed in a direction orthogonal to the axial direction X of the casing 120 and directed toward the bottom inner surface 127. The bottom outer surface 126 includes the groove inner side surfaces 220 and the ceiling surface 222, which are the inner surfaces of the recessed groove 132.

As illustrated in Figs. 3 and 6, the drug solution administration device 100 includes the flow path forming member 166 accommodated within the recessed groove 132. The flow path forming member 166 has the hollow interior 170. The hollow interior 170, together with the recessed groove 132, forms the drug solution flow path 210 through which the drug solution discharged from the reservoir 102 flows.

According to this configuration, it is possible to supply the drug solution from the reservoir 102 inside the casing 120 to the drug solution flow path 210 formed outside the casing 120. In this case, it is unnecessary to bridge a tube or the like from the reservoir 102 to the cannula 66 inside the casing 120. Therefore, it is unnecessary to accommodate a tube inside the casing 120. As a result, the casing 120 can be downsized, thereby enabling the downsizing of the drug solution administration device 100.

Further, in assembling the drug solution administration device 100, it is easier to assemble the flow path forming member 166 outside the casing 120 than to additionally accommodate a tube inside the casing 120 that has already accommodated the reservoir 102 and the pump unit 110.

In the thickness direction T, which is orthogonal to the axial direction (axial direction X) of the casing 120 and directed toward the bottom outer surface 126, the entire flow path forming member 166 is accommodated within the recessed groove 132.

As illustrated in Figs. 7A to 10B, when the drug solution administration device 100 is mounted to or detached from the holder 20, interference of the flow path forming member 166 with the holder 20 is avoided. Accordingly, the drug solution administration device 100 can be smoothly mounted to or detached from the holder 20.

As illustrated in Fig. 5, the reservoir 102 has the drug solution outlet 106 from which the drug solution is discharged. The casing 120 has the communication hole 202 that communicates with the drug solution outlet 106. The drug solution outlet 106 and the drug solution flow path 210 communicate with each other via the communication hole 202.

Inside the casing 120, it is not necessary to establish communication between the drug solution outlet 106 of the reservoir 102 and the hollow interior 170 of the flow path forming member 166 via a tube or the like. Therefore, the number of components inside the casing 120 is reduced, allowing for greater flexibility in the internal layout of the components in the casing 120. Furthermore, the downsizing and weight reduction of the drug solution administration device 100 can also be achieved.

As illustrated in Fig. 4, when the drug solution administration device 100 is viewed in the thickness direction T, at least a portion of the drug solution flow path 210 overlaps with the reservoir 102. In a typical example, the first straight portion 165 of the drug solution flow path 210 overlaps with one end portion of the reservoir 102 in the width direction W. The one end portion of the reservoir 102 in the width direction W and the first straight portion 165 of the drug solution flow path 210 overlap from the end portion on the connection direction X1 side of the reservoir 102 to the end portion on the detachment direction X2 side of the reservoir 102.

According to this configuration, the dimension of the drug solution administration device 100 in the width direction W can be reduced.

The reservoir 102 has the arcuate curved side surface 186 that curves in a convex shape toward the flow path forming member 166. When the direction in the thickness direction T toward the bottom outer surface 126 is defined as the downward direction T2, and the direction opposite to the downward direction T2 and away from the bottom outer surface 126 is defined as the upward direction T1, the position of the flow path forming member 166 in the thickness direction T is between the lowermost portion 188 of the reservoir 102 and the central portion 190 of the reservoir 102 in the thickness direction T. Accordingly, when the drug solution administration device 100 is viewed in the width direction W orthogonal to the axial direction X and the thickness direction T, the first straight portion 165 of the flow path forming member 166 is positioned between (overlaps with) the lowermost portion 188 and the central portion 190.

As a result, the drug solution flow path 210 can be arranged in close proximity to the curved side surface 186. Accordingly, dead space around the reservoir 102 is reduced. Therefore, the drug solution administration device 100 can be downsized.

In a preferred embodiment, the material of the flow path forming member 166 is resin.

In the used drug solution administration system 10, the portion including the reservoir 102 and the flow path forming member 166 is discarded as medical waste. Since the flow path forming member 166 is not made of metal, the environmental burden can be reduced.

In a preferred embodiment, the flow path forming member 166 is transparent.

The user can visually observe the hollow interior 170 of the flow path forming member 166 from the outside. Accordingly, the user can easily confirm by visual inspection whether foreign matter is mixed into the drug solution flowing through the drug solution flow path 210, whether the drug solution has entrained air, or the like.

As understood from Figs. 3 and 6, the drug solution flow path 210 extends in the axial direction of the reservoir 102 (the axial direction X of the drug solution administration device 100). The axial direction of the casing 120 coincides with the axial direction of the reservoir 102.

In this case, compared to a case where the drug solution flow path 210 extends in another direction, the space required to form the drug solution flow path 210 can be reduced. In other words, space-saving of the drug solution flow path 210 is achievable.

The flow direction of the drug solution in the drug solution flow path 210 is opposite to the pushing direction on the drug solution stored inside the reservoir 102.

In this case, compared to a case where the pushing direction on the drug solution stored inside the reservoir 102 coincides with the flow direction of the drug solution in the drug solution flow path 210, the length of the drug solution administration device 100 in the axial direction X can be reduced.

The flow path forming member 166 is substantially L-shaped.

As a result, the flow path forming member 166 can be arranged at a position other than below the lowermost portion 188 of the reservoir 102. Moreover, it is possible to change the direction of the drug solution discharged from the reservoir 102 to a direction opposite to the pushing direction of the gasket 108 on the drug solution, while shortening the length of the drug solution flow path 210. In this case, the direction in which the drug solution administration device 100 is mounted to the holder 20 coincides with the direction in which the second connecting portion 168 is connected to the first connecting portion 74, which is the connection portion of the cannula unit 60. Therefore, in a state when the drug solution administration device 100 is not mounted to the holder 20, the first connecting portion 74 can be reliably sealed with the diaphragm 78. When the drug solution administration device 100 has been mounted to the holder 20, the configuration of connecting the second connecting portion 168 to the first connecting portion 74 can be easily achieved.

As illustrated in Figs. 1 and 3, the drug solution administration system 10 includes: the drug solution administration device 100; and the holder 20 to which the drug solution administration device 100 is detachably mounted. The holder 20 has the cannula unit 60, which includes the cannula 66 to be indwelled in the body BD of the living subject and the housing 62. The cannula unit 60 is positioned in the holder 20 between the two end portions in the axial direction X, and also between the two end portions in the width direction W that is orthogonal to both the axial direction X and the thickness direction T.

Since the cannula unit 60 is not positioned at an edge portion of the holder 20, the entire edge portion of the holder 20 is fixed to the body BD of the patient. Accordingly, the holder 20 is less likely to be detached from the body BD. As a result, detachment of the cannula 66 from the skin SK is avoided.

The cannula unit 60 includes the first connecting portion 74. The flow path forming member 166 includes the second connecting portion 168 that is detachably connected to the first connecting portion 74, and the opening 172 that is formed on a side portion facing the ceiling surface 222 of the recessed groove 132 and communicates with the hollow interior 170. The recessed groove 132 and the hollow interior 170 form the drug solution flow path 210 through which the drug solution discharged from the reservoir 102 flows toward the cannula 66.

Thus, by connecting the first connecting portion 74 of the holder 20 and the second connecting portion 168 of the drug solution administration device 100, the lumen of the cannula 66 can be brought into communication with the drug solution flow path 210 of the drug solution administration device 100. Therefore, the drug solution in the reservoir 102 can be administered to the living subject via the lumen of the cannula 66.

Note that, the present invention is not limited to the above disclosure, and various configurations can be adopted without departing from the gist of the present invention.

## Claims

1. A drug solution administration device comprising: a reservoir that is filled with a drug solution; and a casing that accommodates the reservoir, wherein
the casing has a bottom portion including: a bottom outer surface which faces a living subject when the drug solution is administered to the living subject, and a bottom inner surface which is a back surface of the bottom outer surface, the reservoir is arranged on the bottom inner surface,
the bottom outer surface has a recessed groove which is recessed in a direction orthogonal to an axial direction of the casing and directed toward the bottom inner surface, the bottom outer surface includes a groove inner side surface and a ceiling surface which are inner surfaces of the recessed groove,
the drug solution administration device includes a flow path forming member which is accommodated in the recessed groove and has a hollow interior, and
the hollow interior of the flow path forming member forms, within the recessed groove, a drug solution flow path through which the drug solution flows.

2. The drug solution administration device according to claim 1, wherein an entirety of the flow path forming member is accommodated in the recessed groove in a thickness direction which is orthogonal to the axial direction of the casing and directed toward the bottom outer surface.

3. The drug solution administration device according to claim 1, wherein the reservoir has a drug solution outlet from which the drug solution is discharged, the casing has a communication hole which communicates with the drug solution outlet, and
the drug solution outlet and the drug solution flow path communicate with each other via the communication hole.

4. The drug solution administration device according to claim 1, wherein, when the drug solution administration device is viewed in a thickness direction which is orthogonal to the axial direction of the casing and directed toward the bottom outer surface, at least a portion of the drug solution flow path overlaps with the reservoir.

5. The drug solution administration device according to claim 4, wherein the reservoir has a curved side surface which is arcuate and curves in a convex shape toward the flow path forming member, and
when a direction toward the bottom outer surface in the thickness direction is defined as a downward direction, and a direction opposite to the downward direction and away from the bottom outer surface is defined as an upward direction, a position of the flow path forming member in the thickness direction is between a lowermost portion of the reservoir and a central portion of the reservoir in the thickness direction.

6. The drug solution administration device according to claim 1, wherein a material of the flow path forming member is resin.

7. The drug solution administration device according to claim 6, wherein the flow path forming member is transparent.

8. The drug solution administration device according to any one of claims 1 to 7, wherein the drug solution flow path extends in an axial direction of the reservoir, and the axial direction of the casing coincides with the axial direction of the reservoir.

9. The drug solution administration device according to claim 8, wherein a flow direction of the drug solution in the drug solution flow path is opposite to a pushing direction on the drug solution stored inside the reservoir.

10. The drug solution administration device according to claim 9, wherein the flow path forming member is substantially L-shaped.

11. A drug solution administration system comprising: the drug solution administration device according to claim 1; and a holder that the drug solution administration device is detachably mounted to, wherein
the holder has a cannula unit including a cannula to be indwelled in a body of a living subject, and
the cannula unit is positioned in the holder between two end portions in the axial direction, and between two end portions in a width direction which is orthogonal to both the axial direction and a thickness direction, which is orthogonal to the axial direction of the casing and directed toward the bottom outer surface.

12. The drug solution administration system according to claim 11, wherein the cannula unit has a first connecting portion,
the flow path forming member has: a second connecting portion which is detachably connected to the first connecting portion, and an opening which is formed on a side portion facing the ceiling surface of the recessed groove and communicates with the hollow interior, and
the recessed groove and the hollow interior form a drug solution flow path through which the drug solution discharged from the reservoir flows toward the cannula.
